# EUROPEAN PATENT APPLICATION

(11) **EP 1 577 349 A1**
(43) Date of publication of application: **21.09.2005**
(21) Application number: 03775852.1
(22) Date of filing: 21.11.2003
(51) Int. Cl.: C08L 101/14, C08K 3/00, C08K 5/00

(54) **WATER-ABSORBING RESIN COMPOSITION**

(30) Priority: 27.12.2002 JP 2002381202
(71) Applicant: SUMITOMO SEIKA CHEMICALS CO., LTD., Hyogo 675-0145 (JP)
(72) Inventor: HANDA, Masayoshi Funct.Polymers, Res.Lab, Sumitomo, Himeji-shi, Hyogo 672-8076 (JP); NAWATA, Yasuhiro Funct. Polymers, Res.Lab, Shikama-ku, Himeji-shi, Hyogo 672-8076 (JP)
(74) Representative: Vossius & Partner
(86) International application number: PCT/JP2003/014865
(87) International publication number: WO 2004/061010

(57) **Abstract**

A water-absorbent resin composition comprising an oxygen-containing reducing inorganic salt and a water-absorbent resin, wherein the iron content in the resin composition is at most 1 ppm; an absorbent having the above-mentioned water-absorbent resin composition and a hydrophilic fiber; and an absorbent article comprising the above-mentioned absorbent interposed between a liquid-permeable sheet and a liquid-impermeable sheet.

## Description

### TECHNICAL FIELD

The present invention relates to a water-absorbent resin composition. More specifically, the present invention relates to a water-absorbent resin composition, and an absorbent and an absorbent article in which the water-absorbent resin composition is used. The absorbent article can be suitably used as hygienic materials such as paper diaper, sanitary napkin and incontinence pad; urine-absorbing materials for pets; materials for civil engineering and construction such as packing materials; food freshness retaining materials such as drip absorbents and cold-reserving agents; horticultural articles such as water-retaining materials for soils; and the like.

### BACKGROUND ART

A water-absorbent article such as paper diaper or sanitary napkin has been generally formed by interposing an absorbent composed of a hydrophilic fiber and a water-absorbent resin between a liquid-permeable sheet to be placed on the side contacting a body and a liquid-impermeable sheet to be placed on the side opposite to the liquid-permeable sheet.

In recent years, an absorbent, or paper diaper, sanitary napkin or the like in which the absorbent is used tends to be thinner in order to achieve carrying convenience and a comfortable fit. Along with this thinning, there has been studied the development of an absorbent having a decreased amount of hydrophilic fiber and an increased amount of a water-absorbent resin in order to reduce re-wet or liquid leakage even when the liquid is absorbed in a large amount.

However, since a gel formed by allowing a water-absorbent resin to absorb a body fluid such as human urine generally deteriorates in accordance with the passage of time, its absorbability is lowered. In addition, since the water-absorbent resin itself is partially decomposed as the deterioration of the gel is further progressed, a water-soluble substance is exuded from the gel.

Therefore, when a manufactured article produced by using water-absorbent resins mentioned above is used in contact with a human body, the exuded water-soluble substance is deposited to skin, so that there is a possibility to arise a rash or the like.

Further, in an absorbent containing a water-absorbent resin in a large amount, gel deteriorates with water-absorbent resins closely approaching to each other. Therefore, a phenomenon, a so-called "gel blocking" in which the exuded water-soluble substance clogs a gap between gel particles to prevent the liquid from being permeated into the water-absorbent resin is likely to take place. This gel blocking is considered to be one of the factors for liquid leakage of the absorbent.

Although it is difficult to specify the causation because the mechanism of causing deterioration of the gel is complicated, it is deduced that an iron content in human urine and an iron content in a water-absorbent resin are related thereto as one of the causations.

Accordingly, there have been proposed a water-absorbent resin composition containing an oxygen-containing reducing inorganic salt (Japanese Patent Laid-Open No. Sho 63-118375), a water-absorbent resin composition containing a radical chain inhibitor and a metal chelating agent (Japanese Patent Laid-Open No. Hei 1-210463), and the like in order to improve the stability of a gel.

There is, however, a disadvantage in these water-absorbent resin compositions that sufficient stability of a gel cannot be exhibited when the water-absorbent resin is contained in an absorbent in a large amount.

In addition, since the water-absorbent resin is partially degenerated when the water-absorbent resin is stored for a long period of time in an atmosphere having high temperatures and high humidity, discoloration may occur in some cases. When the water-absorbent resin is discolored, the external appearance of an absorbent and an absorbent article in which the water-absorbent resin is used is impaired, thereby resulting in lowering of the value of a manufactured article. Especially, in an absorbent and an absorbent article in which the water-absorbent resin is contained in a large amount, discoloration of the water-absorbent resin is likely to be more noticeable.

Accordingly, in recent years, there has been desired an improvement in discoloration resistance of a water-absorbent resin. Although it is difficult to specify the causation of the discoloration since the mechanism of discoloration of the water-absorbent resin is very complicated as in the above-mentioned mechanism of causing deterioration or decomposition of the gel, it is deduced that an iron content in the water-absorbent resin is related to the discoloration as one of the causations.

As the water-absorbent resin having improved discoloration resistance, there have been known a water-absorbent resin composition comprising a water-absorbent resin and an organic carboxylic acid and/or its salt (Japanese Patent Laid-Open No. 2000-327926), a water-absorbent resin composition containing a coloring preventive and/or an antioxidant and/or a boron-containing compound (Japanese Patent Laid-Open No. 2000-230129), and the like.

There are, however, some disadvantages in these compositions, such that satisfactory discoloration resistance cannot be imparted to an absorbent containing a water-absorbent resin in a large amount.

Accordingly, there has been earnestly desired to develop a water-absorbent resin composition which is excellent in stability of a gel to a body fluid such as human urine even when an absorbent contains an water-absorbent resin in a large amount, and also excellent in discoloration resistance even at high temperatures and high humidity, an absorbent and an absorbent article in which the water-absorbent resin composition is used.

### DISCLOSURE OF INVENTION

An object of the present invention is to provide a water-absorbent resin composition which is excellent in stability of a gel to a body fluid such as human urine even when the absorbent contains the water-absorbent resin in a large amount, and excellent in discoloration resistance even at high temperatures and high humidity, an absorbent and an absorbent article in which the water-absorbent resin composition is used.

The present invention relates to:
(1) a water-absorbent resin composition comprising an oxygen-containing reducing inorganic salt and a water-absorbent resin, wherein the iron content in the resin composition is at most 1 ppm;
(2) the above-mentioned water-absorbent resin composition, further comprising an organic antioxidant;
(3) an absorbent comprising the above-mentioned water-absorbent resin composition and a hydrophilic fiber; and
(4) an absorbent article comprising the above-mentioned absorbent interposed between a liquid-permeable sheet and a liquid-impermeable sheet.

### DETAILED DESCRIPTION OF THE INVENTION

One of the great features of the water-absorbent resin composition of the present invention resides in that the water-absorbent resin composition comprises an oxygen-containing reducing inorganic salt and a water-absorbent resin, and that the iron content in the resin composition is at most 1 ppm. Since the water-absorbent resin composition of the present invention has this feature, there are exhibited some excellent effects such that the water-absorbent resin composition is excellent in stability of a gel to a body fluid such as human urine even when an absorbent contains the water-absorbent resin in a large amount, and excellent in discoloration resistance even at high temperatures and high humidity.

In addition, when the water-absorbent resin composition of the present invention further comprises an organic antioxidant, there are exhibited some excellent effects such that the water-absorbent resin composition is excellent in stability of a gel to not only a body fluid such as human urine but also an electrolytic aqueous solution, and excellent in discoloration resistance even at high temperatures and high humidity.

The water-absorbent resin used in the present invention includes, for instance, crosslinked polymers of acrylic acid salt, crosslinked hydrolysates of starch-acrylic acid salt graftcopolymers, crosslinked copolymers of vinyl alcohol-acrylic acid salt, crosslinked maleic anhydride-grafted polyvinyl alcohol, crosslinked isobutylene-maleic anhydride copolymers, partially neutralized crosslinked polyacrylic acid, saponified vinyl acetate-acrylic ester copolymers, and the like. Among them, the crosslinked polymers of acrylic acid salt is preferable since the polymer is capable of absorbing water in a large amount and retaining the absorbed water in its molecule even when a certain load is applied to the polymer.

It is desired that the iron content in the water-absorbent resin is at most 1 ppm, preferably at most 0.5 ppm from the viewpoint of lowering the iron content in the resulting water-absorbent resin composition.

The process for preparing a water-absorbent resin is not limited to specified one. Representative preparation processes thereof include a process for preparing a water-absorbent resin from a raw material having a lower iron content according to a reversed phase suspension polymerization method, an aqueous solution polymerization method and the like.

The "iron content" as used herein refers to a value obtained when the content of iron in a sample is determined in accordance with the following determination method. In the present invention, as the sample, there can be specifically used a water-absorbent resin, an oxygen-containing reducing inorganic salt, an organic antioxidant, or a water-absorbent resin composition.

### [Determination Method of Iron Content]

One gram of a sample is accurately weighed, and placed in a 200 mL beaker. Three milliliters of concentrated sulfuric acid and 5 mL of concentrated nitric acid are added to the beaker to sufficiently dissolve the sample. The solution obtained is heated to a temperature near its boiling point to decompose the sample.

Then, the resulting black liquid is continued to be heated with adding nitric acid thereto under stirring until the black liquid becomes transparent. The resulting transparent liquid is further heated at 350°C, thereby evaporating the transparent liquid to dryness to collect solids.

The solids obtained are cooled to 25°C, and thereafter the total amount of the solids and 1 mL of concentrated hydrochloric acid are placed in a 50 mL volumetric flask. Distilled water is added thereto so as to make up the total amount of 50 mL to give a sample solution.

Absorbance of the resulting sample solution is determined with an atomic absorption photometer (manufactured by Shimadzu Corporation under the product number of AA-6700).

Separately from the above, absorbance of an iron standard solution is determined in the same manner as in the above to previously draw a calibration curve. The iron content (ppm) in the sample is calculated from the calibration curve.

The oxygen-containing reducing inorganic salt used in the present invention includes, for instance, sulfites such as sodium sulfite, potassium sulfite, calcium sulfite, zinc sulfite and ammonium sulfite; bisulfites such as sodium bisulfite, potassium bisulfite, calcium bisulfite and ammonium bisulfite; pyrosulfites such as sodium pyrosulfite, potassium pyrosulfite and ammonium pyrosulfite; dithionites such as sodium dithionite, potassium dithionite, ammonium dithionite, calcium dithionite and zinc dithionite; trithionates such as potassium trithionate and sodium trithionate; tetrathionates such as potassium tetrathionate and sodium tetrathionate; thiosulfates such as sodium thiosulfate, potassium thiosulfate and ammonium thiosulfate; nitrites such as sodium nitrite, potassium nitrite, calcium nitrite and zinc nitrite; and the like. Among them, the sulfites, the bisulfites, the pyrosulfites, the dithionites and the nitrites are preferable, the sulfites and the bisulfites are more preferable, and sodium sulfite, potassium sulfite, sodium bisulfite and potassium bisulfite are even more preferable from the viewpoint of enhancing stability of a gel and discoloration resistance.

It is desired that the iron content in the oxygen-containing reducing inorganic salt is at most 10 ppm, preferably at most 5 ppm, more preferably at most 3 ppm from the viewpoint of lowering the iron content in the resulting water-absorbent resin composition.

It is desired that the amount of the oxygen-containing reducing inorganic salt is at least 0.01 parts by weight, preferably at least 0.05 parts by weight based on 100 parts by weight of the water-absorbent resin from the viewpoint of enhancing stability of a gel and discoloration resistance. In addition, even when the oxygen-containing reducing inorganic salt is used in an exceedingly large amount, not only the stability of a gel and discoloration resistance corresponding to the amount are not exhibited, thereby making it uneconomical, but also the iron content in the water-absorbent resin composition is increased. Therefore, it is desired that the amount of the oxygen-containing reducing inorganic salt is at most 5 parts by weight, preferably at most 3 parts by weight based on 100 parts by weight of the water-absorbent resin. Accordingly, it is desired that the amount of the oxygen-containing reducing inorganic salt is 0.01 to 5 parts by weight, preferably 0.05 to 3 parts by weight based on 100 parts by weight of the water-absorbent resin in view of these matters.

The water-absorbent resin composition of the present invention can be obtained by, for instance, mixing the water-absorbent resin with the oxygen-containing reducing inorganic salt. Its mixing process is not limited to specified ones.

Examples of the process for mixing a water-absorbent resin with an oxygen-containing reducing inorganic salt include (i) a process comprising adding an oxygen-containing reducing inorganic salt to an aqueous solution of a monomer before polymerization, which constitutes a water-absorbent resin, and mixing them; (ii) a process comprising adding an oxygen-containing reducing inorganic salt to a water-containing gel-like product of a water-absorbent resin, and mixing them; (iii) a process comprising adding an oxygen-containing reducing inorganic salt to a water-absorbent resin while or after drying the water-absorbent resin, and mixing them; and the like. Among these processes, the above-mentioned process (iii) is preferable since the procedures are simple and convenient.

The oxygen-containing reducing inorganic salt can be used in any form of powder and aqueous solution, as long as stability of a gel is not impaired. It is preferable that the oxygen-containing reducing inorganic salt is powdery, in view of operability during the preparation.

A mixer used when the water-absorbent resin is mixed with the oxygen-containing reducing inorganic salt includes, for instance, a Nauta mixer, a Ribbon blender, a cross-rotary mixer, a conical blender, a double-arm type kneader, a screw-type blender, a V-shaped blender, a W-shaped blender, a turbulizer, and the like. The present invention, however, is not limited only to those exemplified ones.

The iron content in the thus obtained water-absorbent resin composition of the present invention, comprising the oxygen-containing reducing inorganic salt and the water-absorbent resin, is at most 1 ppm, preferably at most 0.5 ppm. When the iron content is adjusted as described above, satisfactory stability and discoloration resistance of a gel can be exhibited.

In the present invention, the above-mentioned water-absorbent resin composition can further comprise an organic antioxidant. As described above, when the organic antioxidant is contained in the water-absorbent resin composition, stability of a gel to an electrolytic aqueous solution can be enhanced.

The organic antioxidant is not limited to specified ones. The organic antioxidant includes, for instance, ascorbic acids such as L-ascorbic acid, sodium L-ascorbate, D-ascorbic acid and sodium D-ascorbate; erythorbic acids such as erythorbic acid and sodium erythorbate; gallic acids such as gallic acid, methyl gallate, ethyl gallate, n-propyl gallate, isoamyl gallate, octyl gallate and lauryl gallate; protocatechuic acids such as protocatechuic acid and ethyl protocatechuate; benzimidazoles such as 2-mercaptobenzimidazole; alkylated hydroxyanisoles such as butylated hydroxyanisole; and the like. These may be used alone or in admixture of at least two kinds. Among them, the ascorbic acids, the erythorbic acids, the gallic acids, the protocatechuic acids, the benzimidazoles and the alkylated hydroxyanisoles are preferable, the ascorbic acids, the erythorbic acids and the gallic acids are more preferable, and L-ascorbic acid, sodium erythorbate and n-propyl gallate are even more preferable because these compounds are excellent in stability of a gel to an electrolytic aqueous solution.

It is desired that the iron content in the organic antioxidant is at most 5 ppm, preferably at most 3 ppm from the viewpoint of lowering the iron content in the resulting water-absorbent resin composition.

It is desired that the amount of the organic antioxidant is at least 0.001 parts by weight, preferably at least 0.005 parts by weight based on 100 parts by weight of the water-absorbent resin from the viewpoint of exhibiting sufficient stability of the gel to the electrolytic aqueous solution. In addition, even when the organic antioxidant is used in a large amount, not only the stability and discoloration resistance of a gel, corresponding to the increased amount are not exhibited, thereby making it uneconomical, but also the iron content in the water-absorbent resin composition increases. Therefore, it is desired that the amount of the organic antioxidant is at most 5 parts by weight, preferably at most 2 parts by weight based on 100 parts by weight of the water-absorbent resin. It is desired that the amount of the organic antioxidant is 0.001 to 5 parts by weight, preferably 0.005 to 2 parts by weight based on 100 parts by weight of the water-absorbent resin in view of these matters.

It is preferred that the organic antioxidant is usually used in combination with the oxygen-containing reducing inorganic salt from the viewpoint of enhancing the stability and discoloration resistance of a gel.

The water-absorbent resin composition comprising the organic antioxidant can be obtained by mixing a water-absorbent resin, an oxygen-containing reducing inorganic salt and an organic antioxidant. The process for mixing these components and the order of adding these components are not limited to specified ones.

Examples of the process for preparing a water-absorbent resin composition comprising an organic antioxidant include (i) a process comprising adding an organic antioxidant to an aqueous monomer solution before polymerization, which constitutes a water-absorbent resin; (ii) a process comprising adding an organic antioxidant to a water-containing gel-like product of a water-absorbent resin; (iii) a process comprising adding an organic antioxidant to a water-absorbent resin while or after drying the water-absorbent resin; and the like. Among these processes, the above-mentioned process (iii) is preferable since the procedures are simple and convenient.

The organic antioxidant can be used in any form of powder and aqueous solution, as long as stability of a gel is not impaired. It is preferable that the organic antioxidant is powdery, in view of operability during the preparation.

A mixer used when the water-absorbent resin is mixed with the oxygen-containing reducing inorganic salt and the organic antioxidant includes, for instance, a Nauta mixer, a Ribbon blender, a cross-rotary mixer, a conical blender, a double-arm type kneader, a screw-type blender, a V-shaped blender, a W-shaped blender, a turbulizer, and the like. The present invention, however, is not limited only to those exemplified ones.

The iron content in the thus obtained water-absorbent resin composition of the present invention, comprising the oxygen-containing reducing inorganic salt, the water-absorbent resin and the organic antioxidant, is at most 1 ppm, preferably at most 0.5 ppm. When the iron content is adjusted as described above, satisfactory stability and discoloration resistance of a gel can be exhibited.

According to the present invention, an absorbent can be obtained by using the above-mentioned water-absorbent resin composition.

The absorbent comprises a water-absorbent resin composition and a hydrophilic fiber.

The hydrophilic fiber is not limited to specified ones. Examples of the hydrophilic fiber include a cellulose fiber, an artificial cellulose fiber, and the like. The hydrophilic fiber may contain a synthetic fiber having hydrophobicity within the range that does not hinder the object of the present invention.

An adhesive binder such as a heat-fusible synthetic fiber, a hot melt adhesive or an adhesive emulsion may be added to the absorbent of the present invention in order to enhance the retention of the shape of the absorbent.

The content of the water-absorbent resin composition in the absorbent is preferably at least 40% by weight, more preferably at least 50% by weight from the viewpoint of sufficiently absorbing a body fluid such as urine and giving a comfortable fit. In addition, the content of the water-absorbent resin composition in the absorbent is preferably at most 95% by weight, more preferably 90% by weight in view of containing the hydrophilic fiber and the adhesive binder therein in order to enhance the retention of the shape of the resulting absorbent. The content of the water-absorbent resin composition in the absorbent is preferably 40 to 95% by weight, more preferably 50 to 90% by weight in view of these matters.

The embodiments for the absorbent of the present invention are not limited to specified ones, as long as the absorbent comprises a water-absorbent resin composition and a hydrophilic fiber.

Preferred embodiments of the absorbent include, for instance, a mixed-type dispersion obtained by mixing a water-absorbent resin composition with a hydrophilic fiber so as to have a uniform composition; a sandwiched-type structure in which a water-absorbent resin composition is interposed between two layers of hydrophilic fibers.

An absorbent article can be produced, for instance, by interposing the above-mentioned absorbent between a liquid-permeable sheet and a liquid-impermeable sheet.

The liquid-permeable sheet is not limited to specified ones, and includes air-through type nonwoven fabrics, spun bond type nonwoven fabrics, chemical bond type nonwoven fabrics, needle-punched type nonwoven fabrics, and the like, which are composed of fibers of polyethylene, polypropylene, polyester or the like.

The liquid-impermeable sheet is not limited to specified ones, and includes synthetic resin films made of a resin such as polyethylene, polypropylene or polyvinyl chloride, and the like.

The kind of the absorbent article is not limited to specified ones. Representative examples of the absorbent article include hygienic materials such as paper diaper, sanitary napkin and incontinence pad; urine-absorbing materials for pets; materials for civil engineering and construction such as packing materials; food freshness retaining materials such as drip absorbents and cold-reserving agents; horticultural articles such as water-retaining materials for soils; and the like.

### EXAMPLES

The present invention will be described more specifically hereinbelow by means of Examples and Comparative Examples, without intending to limit the present invention only to these Examples.

The iron content in each of a water-absorbent resin, an oxygen-containing reducing inorganic salt, an organic antioxidant and a water-absorbent resin composition refers to a value determined by the method described above.

In addition, physical properties of a water-absorbent resin composition and an absorbent article obtained by using the water-absorbent resin composition, which are produced in each Example and each Comparative Example are evaluated in accordance with the following methods.

### (1) Stability of Gel During Absorption of Human Urine

Thirty-nine grams of human urine taken from adult male was added to a 100 mL beaker, and 1 g of a water-absorbent resin composition was added thereto to prepare a human urine-absorbed gel. This human urine-absorbed gel was allowed to stand for 24 hours in an incubator at a temperature of 40°C. Thereafter, stability of the gel was evaluated in accordance with the following evaluation criteria.

### [Evaluation Criteria]

ⓞ: Gel has elasticity and is not crushed even when strongly pressed.
O: Gel has elasticity but is crushed when strongly pressed.
Δ: Gel maintains its shape but is crushed when lightly pinched between fingers.
×: Shape of the gel has collapsed.

### (2) Stability of Gel During Absorption of Physiological Saline

Thirty-nine grams of a 0.9% by weight physiological saline was added in a 100 mL beaker, and 1 g of a water-absorbent resin composition was added thereto to prepare a physiological saline-absorbed gel. This physiological saline-absorbed gel was allowed to stand for 24 hours in an incubator at a temperature of 40°C. Thereafter, stability of the gel was evaluated in accordance with the following evaluation criteria.

### [Evaluation Criteria]

ⓞ: Gel has elasticity and is not crushed even when strongly pressed.
O: Gel has elasticity but is crushed when strongly pressed.
Δ: Gel maintains its shape but is crushed when lightly pinched between fingers.
×: Shape of the gel has collapsed.

### (3) Monitoring Test

Thirteen grams of a water-absorbent resin composition and 7 g of disintergrated wooden pulp were dry-blended, and the mixture was formed to have a size of 40 cm in length and 12 cm in width. Each of the top and bottom of the resulting mixed-type dispersion was interposed between 0.5 g of tissue paper, and a load was applied entirely thereto to prepare an absorbent.

An absorbent inside a commercially available diaper (L size) for babies was completely removed from the side of a liquid-impermeable sheet (backsheet), and the above-mentioned absorbent was then carefully inserted into the diaper. The diaper was sealed with a tape to prepare an absorbent article for monitoring test.

The absorbent articles obtained were used by several children, and thereafter the used absorbent articles were collected.

Next, percentage of defective gel, average absorption amount and leakage percentage of the absorbent articles collected were determined in accordance with the following methods.

### A. Percentage of Defective Gel

The defectiveness of the gel was evaluated in accordance with the following evaluation criteria. The percentage of defective gel was calculated by dividing the number of the absorbent articles found to show defective gel by the number of the absorbent articles collected, and then multiplying the resulting value by a factor of 100.

### [Evaluation Criteria]

×: Shape of the gel has collapsed (defined as defective gel).
O: Shape of the gel has not collapsed.

### B. Average Absorption Amount

The average absorption amount was calculated by dividing an accumulated value of the urine absorption amount of the collected absorbent articles by the number of the absorbent articles collected.

### C. Leakage Percentage

The leakage percentage was calculated by dividing the number of the absorbent articles found to show leakage by the number of the absorbent articles collected, and multiplying the resulting value by a factor of 100.

### (4) Discoloration Resistance of Absorbent Article

Thirteen grams of a water-absorbent resin composition and 7 g of disintergrated wooden pulp were dry-blended, and the mixture was formed to have a size of 40 cm in length and 12 cm in width. Each of the top and bottom of the resulting mixed-type dispersion was interposed between 0.5 g of tissue paper, and a load was applied entirely thereto to prepare an absorbent. This absorbent was interposed between a liquid-permeable polyethylene air-through-type nonwoven fabric having a basis weight of 20 g/m² placed on its top and a liquid-impermeable polyethylene sheet at its bottom, to prepare an absorbent article for discoloration resistance test.

The resulting absorbent article was allowed to stand for 20 days in a thermohygrostat set at a temperature of 50° ± 2°C and a relative humidity of 90 ± 2%. Thereafter, discoloration of the water-absorbent resin composition in the absorbent article was visually observed, and discoloration resistance of the absorbent article was evaluated on the basis of the following evaluation criteria.

### [Evaluation Criteria]

A: Inside of the water-absorbent resin is not discolored when observed after the nonwoven fabric is removed and the absorbent is unweaved.
B: Discoloration of the water-absorbent resin is not found when observed from the top of the nonwoven fabric, but discoloration is found in a part of the water-absorbent resin when the nonwoven fabric is removed and the absorbent is unweaved.
C: Discoloration of the water-absorbent resin is found when observed from the top of the nonwoven fabric.

### Preparation Example 1

Five-hundred milliliters of n-heptane was added to a 1000 mL-five-neck cylindrical round bottomed flask equipped with a stirrer, a reflux condenser, a dropping funnel, a thermometer and a nitrogen gas inlet tube. Thereto was added as a surfactant 0.92 g of a sucrose fatty acid ester having an HLB of 3.0 (manufactured by MITSUBISHI CHEMICAL CORPORATION under the trade name of S-370) to disperse. The temperature of the mixture was raised to dissolve the surfactant, and thereafter cooled to 55°C.

Separately from the above, 92 g of an 80% by weight aqueous acrylic acid solution was added to a 500 mL-Erlenmeyer flask. The amount 102.2 g of a 30% by weight aqueous sodium hydroxide solution (iron content: 0.2 ppm) was added dropwise to this Erlenmeyer flask with externally cooling the mixture to neutralize 75% by mol of acrylic acid, thereby giving a partially neutralized product of acrylic acid. Further, 50.2 g of water, 0.11 g of potassium persulfate as a polymerization initiator, and 9.2 mg of ethylene glycol diglycidyl ether as a crosslinking agent were added thereto to give an aqueous monomer solution for a first step polymerization.

The entire amount of this aqueous monomer solution for a first step polymerization was added to the above-mentioned five-neck cylindrical round bottomed flask with stirring to disperse. The internal of the system was sufficiently replaced with nitrogen gas, and thereafter the temperature of the mixture was raised. The polymerization reaction was carried out for 1 hour with keeping the bath temperature at 70°C. Thereafter, the mixture was cooled to room temperature to give a polymerization slurry.

The amount 119.1 g of an 80% by weight aqueous acrylic acid solution was added to another separate 500 mL-Erlenmeyer flask. Thereto was added dropwise 132.2 g of a 30% by weight aqueous sodium hydroxide solution (iron content: 0.2 ppm) with cooling the mixture, to neutralize 75% by mol of acrylic acid. The amount 27.4 g of water, 0.14 g of potassium persulfate, and 35.7 mg of ethylene glycol diglycidyl ether were added thereto to give an aqueous monomer solution for a second step polymerization. The aqueous solution was cooled in an ice water bath.

The entire amount of this aqueous monomer solution for a second step polymerization was added to the polymerization slurry obtained above. Thereafter, the internal of the system was again sufficiently replaced with nitrogen gas, the temperature of the mixture was then raised, and the second-step polymerization reaction was carried out for 2 hours with keeping the bath temperature at 70°C. After the termination of the polymerization reaction, only water was removed from the water-containing gel-like product being dispersed in n-heptane to the external of the system by azeotropic distillation. To the gel-like product obtained was added 8.44 g of a 2% by weight aqueous solution of ethylene glycol diglycidyl ether, and the mixture was dried by further removing water and n-heptane from the mixture by distillation to give 215.5 g of a water-absorbent resin. The iron content in this water-absorbent resin was 0.3 ppm.

### Preparation Example 2

The same procedures as in Preparation Example 1 were carried out except that a 30% by weight aqueous sodium hydroxide solution (iron content: 0.5 ppm) was used instead of the 30% by weight aqueous sodium hydroxide solution (iron content: 0.2 ppm) in Preparation Example 1 to give 216.9 g of a water-absorbent resin. The iron content in this water-absorbent resin was 0.6 ppm.

### Preparation Example 3

The same procedures as in Preparation Example 1 were carried out except that a 30% by weight aqueous sodium hydroxide solution (iron content: 3.3 ppm) was used instead of the 30% by weight aqueous sodium hydroxide solution (iron content: 0.2 ppm) in Preparation Example 1 to give 216.9 g of a water-absorbent resin. The iron content in this water-absorbent resin was 3.5 ppm.

### Preparation Example 4

The same procedures as in Preparation Example 1 were carried out except that a 30% by weight aqueous sodium hydroxide solution (iron content: 6.5 ppm) was used instead of the 30% by weight aqueous sodium hydroxide solution (iron content: 0.2 ppm) in Preparation Example 1 to give 216.9 g of a water-absorbent resin. The iron content in this water-absorbent resin was 7.0 ppm.

### Example 1

A 2 L-polyethylene container was charged with 100 parts by weight of a water-absorbent resin obtained in the same manner as in Preparation Example 1. Two parts by weight of sodium sulfite A (manufactured by DAITO CHEMICAL CO., LTD. under the trade name of SODIUM METASULFITE for food additives, iron content: 1.4 ppm) was added thereto, and the mixture was mixed for 1 hour in the polyethylene container at an autorotation speed of 30 rpm and a revolution speed of 30 rpm using a cross-rotary mixer (manufactured by MEIWA KOGYO CO., LTD. under the product number of CM-3) to give a water-absorbent resin composition. The iron content in this water-absorbent resin composition was 0.3 ppm.

Thirteen grams of the resulting water-absorbent resin composition and 7 g of disintergrated wooden pulp were dry-blended, and the mixture was formed to have a size of 40 cm in length and 12 cm in width. Each of the top and bottom of the resulting mixed-type dispersion was interposed between 0.5 g of tissue paper, and a load was applied entirely thereto to prepare an absorbent.

An absorbent inside a commercially available diaper (L size) for babies was completely removed from the side of a liquid-impermeable sheet (backsheet), and the above-mentioned absorbent was then carefully inserted into the diaper. The diaper was sealed with a tape to prepare an absorbent article for monitoring test.

On the other hand, the above-mentioned absorbent was interposed between a liquid-permeable polyethylene air-through-type nonwoven fabric having a basis weight of 20 g/m² placed on its top and a liquid-impermeable polyethylene sheet at its bottom, to prepare an absorbent article for discoloration resistance test.

As the physical properties of the resulting water-absorbent resin composition, (1) stability of gel during absorption of human urine and (2) stability of gel during absorption of physiological saline were evaluated in accordance with the above-mentioned methods. In addition, (3) monitoring test described above was carried out using the absorbent article for monitoring test. Moreover, (4) discoloration resistance of absorbent article described above was evaluated by the absorbent article for discoloration resistance test. The results are shown in Table 2.

### Examples 2 to 6 and Comparative Examples 1 to 3

The same procedures as in Example 1 were carried out except that 100 parts by weight of the water-absorbent resin obtained in Preparation Example No. shown in Table 1 was used as a water-absorbent resin, and that the oxygen-containing reducing inorganic salt shown in Table 1 was used as an oxygen-containing reducing inorganic salt in an amount shown in Table 1 in Example 1, to give a water-absorbent resin composition. The iron content in the water-absorbent resin composition obtained is shown in Table 1.

Details of the oxygen-containing reducing inorganic salt listed in Table 1 and Table 3 given later are as follows:
Sodium Sulfite A: manufactured by DAITO CHEMICAL CO., LTD under the trade name of SODIUM METASULFITE for food additives, iron content: 1.4 ppm
Sodium Sulfite B: manufactured by DAITO CHEMICAL CO., LTD under the trade name of SODIUM METASULFITE (ANHYDROUS) 90, iron content: 23 ppm
Sodium Bisulfite A: manufactured by DAITO CHEMICAL CO., LTD under the trade name of SODIUM METABISULFITE, iron content: 1.5 ppm
Sodium Bisulfite B: manufactured by KANTO KAGAKU under the trade name of SODIUM BISULFITE, FIRST GRADE, iron content: 15 ppm
Potassium Pyrosulfite: manufactured by DAITO CHEMICAL CO., LTD under the trade name of POTASSIUM PYROSULFITE, iron content: 1.3 ppm
Sodium Dithionite: manufactured by NISSAN CHEMICAL INDUSTRIES, LTD. under the trade name of SODIUM HYDROSULFITE for food additives, iron content: 1.9 ppm
Sodium Nitrite: manufactured by NISSAN CHEMICAL INDUSTRIES, LTD. under the trade name of SODIUM NITRITE, iron content: 2.5 ppm

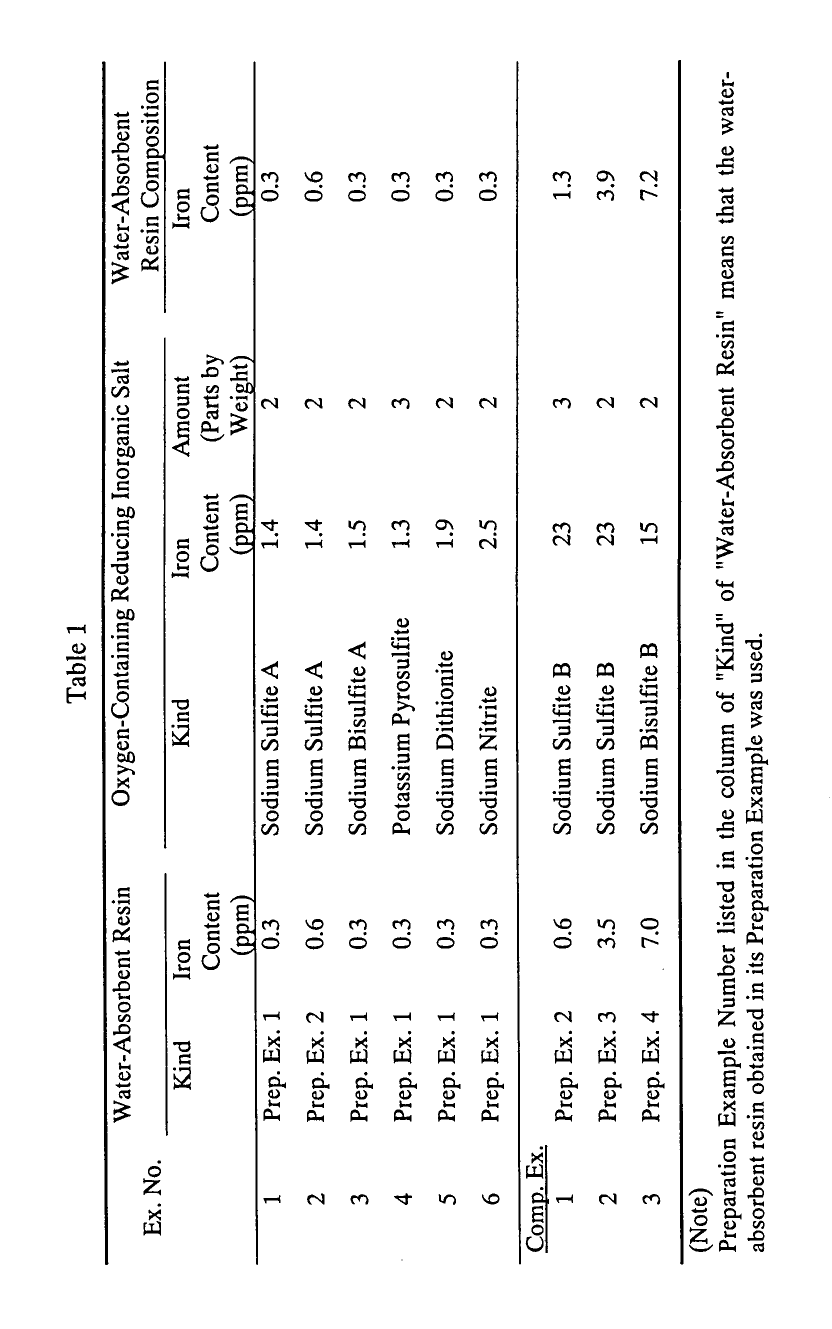

Next, an absorbent and an absorbent article were produced by using the water-absorbent resin composition obtained in each Example or each Comparative Example in the same manner as in Example 1, and their physical properties were evaluated. The results are shown in Table 2.

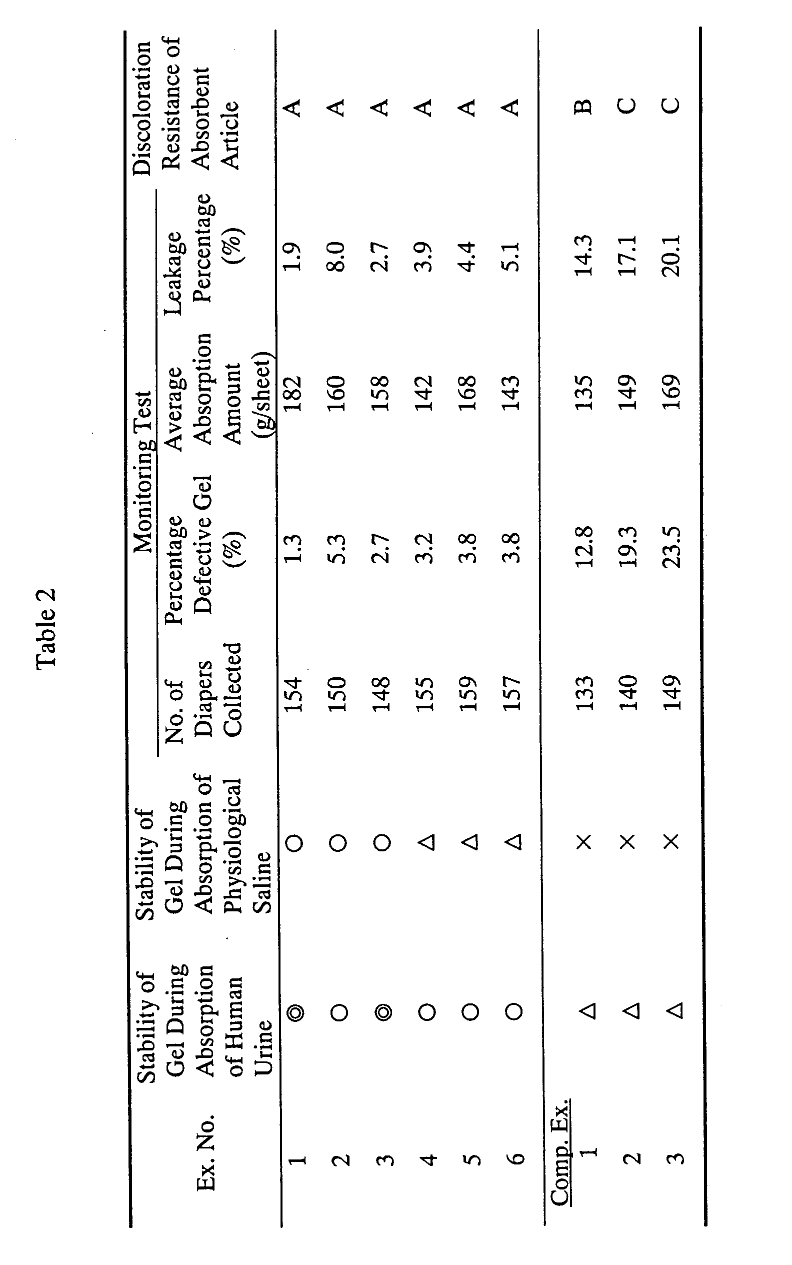

It can be seen from the results shown in Table 2 that according to each Example, there is obtained a water-absorbent resin composition which is excellent in stability of a gel to a body fluid such as human urine even when the absorbent contains a water-absorbent resin in a large amount, and excellent in discoloration resistance even at high temperatures and high humidity.

### Example 7

A 2 L-polyethylene container was charged with 100 parts by weight of a water-absorbent resin obtained in the same manner as in Preparation Example 1. Two parts by weight of sodium sulfite (manufactured by DAITO CHEMICAL CO., LTD under the trade name of SODIUM METASULFITE for food additives, iron content: 1.4 ppm) and 0.02 parts by weight of L-ascorbic acid (manufactured by Roche Vitamin Japan KK under the trade name of L-ASCORBIC ACID, iron content: 0.8 ppm) were added thereto, and the mixture was mixed for 1 hour in the polyethylene container at an autorotation speed of 30 rpm and a revolution speed of 30 rpm using a cross-rotary mixer (manufactured by MEIWA KOGYO CO., LTD. under the product number of CM-3) to give a water-absorbent resin composition. The iron content in the resulting water-absorbent resin composition is shown in Table 3.

Thirteen grams of the resulting water-absorbent resin composition and 7 g of disintergrated wooden pulp were dry-blended, and the mixture was formed to have a size of 40 cm in length and 12 cm in width. Each of the top and bottom of the resulting mixed-type dispersion was interposed between 0.5 g of tissue paper, and a load was applied entirely thereto to prepare an absorbent.

An absorbent inside a commercially available diaper (L size) for babies was completely removed from the side of a liquid-impermeable sheet (backsheet), and the above-mentioned absorbent was then carefully inserted into the diaper. The diaper was sealed with a tape to prepare an absorbent article for monitoring test.

On the other hand, the above-mentioned absorbent was interposed between a liquid-permeable polyethylene air-through-type nonwoven fabric having a basis weight of 20 g/m² placed on its top and a liquid-impermeable polyethylene sheet at its bottom, to prepare an absorbent article for discoloration resistance test.

As the physical properties of the resulting water-absorbent resin composition, (1) stability of gel during absorption of human urine and (2) stability of gel during absorption of physiological saline were evaluated in accordance with the above-mentioned methods. In addition, (3) monitoring test described above was carried out by the absorbent article for monitoring test. Moreover, (4) discoloration resistance of absorbent article described above was evaluated by the absorbent article for discoloration resistance test. The results are shown in Table 4.

### Examples 8 to 13 and Comparative Examples 4 to 6

The same procedures as in Example 7 were carried out except that 100 parts by weight of the water-absorbent resin obtained in Preparation Example No. shown in Table 3 was used as a water-absorbent resin, and that the oxygen-containing reducing inorganic salt and the organic antioxidant shown in Table 3 were used as an oxygen-containing reducing inorganic salt and an organic antioxidant in an amount shown in Table 3 in Example 7, to give a water-absorbent resin composition. The iron content in the resulting water-absorbent resin composition is shown in Table 3.

Details of the organic antioxidant listed in Table 3 are as follows:
L-Ascorbic Acid A: manufactured by Roche Vitamin Japan KK under the trade name of L-ASCORBIC ACID, iron content: 0.8 ppm
n-Propyl Gallate A: manufactured by DAINIPPON PHARMACEUTICAL CO., LTD. under the trade name of n-PROPYL GALLATE for food additives, iron content: 0.4 ppm
n-Propyl Gallate B: manufactured by Midori Kagaku Co., Ltd. under the trade name of n-PROPYL GALLATE for feed, iron content: 5.5 ppm
Sodium Erythorbate: manufactured by Fujisawa Pharmaceutical Co., Ltd. under the trade name of SODIUM ERYTHORBATE, iron content: 1.2 ppm 2-Mercaptobenzimidazole: manufactured by OUCHI SHINKO CHEMICAL INDUSTRIAL CO., LTD. under the trade name of NOCRAC MB, iron content: 1.8 ppm
Ethyl Protocatechuate: manufactured by KANTO KAGAKU under the trade name of ETHYL PROTOCATECHUATE, iron content: 1.8 ppm
Butylated Hydroxyanisole: manufactured by Wako Pure Chemical Industries, Ltd. under the trade name of BUTYLATED HYDROXYANISOLE, iron content: 2.0 ppm
L-Ascorbic Acid B: manufactured by Wako Pure Chemical Industries, Ltd. under the trade name of L-ASCORBIC ACID, FIRST GRADE, iron content: 6.2 ppm

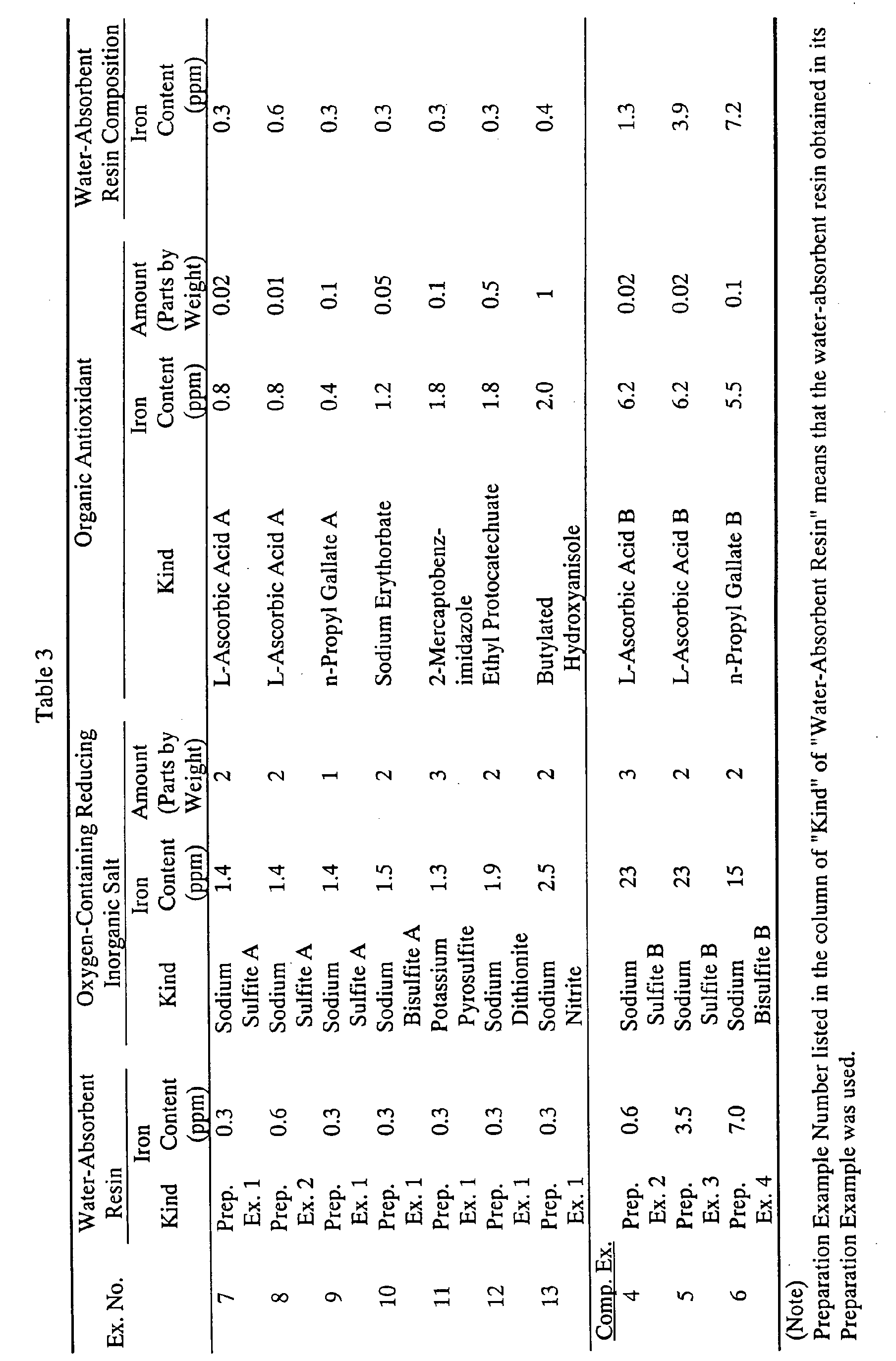

Next, an absorbent and an absorbent article were prepared by using the resulting water-absorbent resin composition in the same manner as in Example 7, and their physical properties were evaluated. The results are shown in Table 4.

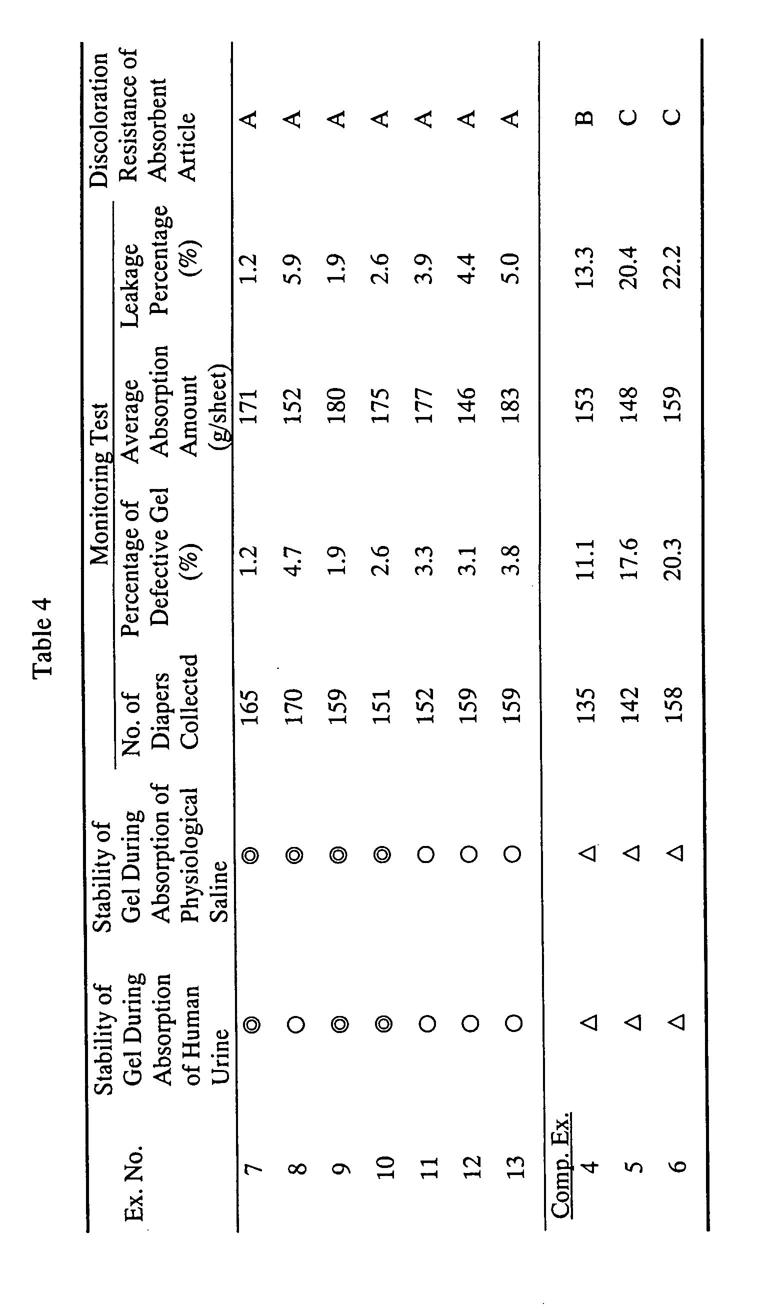

It can be seen from the results shown in Table 4 that according to each Example, there is obtained a water-absorbent resin composition which is excellent in stability of a gel to a body fluid such as human urine even when the absorbent contains a water-absorbent resin in a large amount, excellent in stability of a gel to an electrolytic aqueous solution such as physiological saline, and excellent in discoloration resistance even at high temperatures and high humidity.

As explained above, the water-absorbent resin composition of the present invention is excellent in stability of a gel to a body fluid such as human urine even when the absorbent contains a water-absorbent resin in a large amount, and excellent in discoloration resistance even at high temperatures and high humidity. Therefore, when the water-absorbent resin composition is used, an absorbent and an absorbent article being excellent in these physical properties can be obtained.

### INDUSTRIAL APPLICABILITY

An absorbent and an absorbent article, in which the water-absorbent resin composition of the present invention is used can be suitably used as hygienic materials such as paper diaper, sanitary napkin, and incontinence pad; urine-absorbing materials for pets; materials for civil engineering and construction such as packing materials; food freshness retaining materials such as drip absorbents and cold-reserving agents; horticultural articles such as water-retaining materials for soils; and the like.

## Claims

1. A water-absorbent resin composition comprising an oxygen-containing reducing inorganic salt and a water-absorbent resin, wherein the iron content in the resin composition is at most 1 ppm.

2. The water-absorbent resin composition according to claim 1, wherein the oxygen-containing reducing inorganic salt is at least one member selected from the group consisting of sulfites, bisulfites, pyrosulfites, dithionites and nitrites.

3. The water-absorbent resin composition according to claim 1 or 2, wherein the amount of the oxygen-containing reducing inorganic salt is 0.01 to 5 parts by weight based on 100 parts by weight of the water-absorbent resin.

4. The water-absorbent resin composition according to any one of claims 1 to 3, further comprising an organic antioxidant.

5. The water-absorbent resin composition according to claim 4, wherein the organic antioxidant is at least one member selected from the group consisting of ascorbic acids, erythorbic acids, gallic acids, protocatechuic acids, benzimidazoles and alkylated hydroxyanisoles.

6. The water-absorbent resin composition according to claim 4 or 5, wherein the amount of the organic antioxidant is 0.001 to 5 parts by weight based on 100 parts by weight of the water-absorbent resin.

7. An absorbent comprising the water-absorbent resin composition as defined in any one of claims 1 to 6 and a hydrophilic fiber.

8. The absorbent according to claim 7, wherein the content of the water-absorbent resin composition in the absorbent is 40 to 95% by weight.

9. An absorbent article comprising the absorbent as defined in claim 7 or 8 interposed between a liquid-permeable sheet and a liquid-impermeable sheet.
